# EUROPEAN PATENT APPLICATION

(11) **EP 1 582 596 A2**
(43) Date of publication of application: **05.10.2005**
(21) Application number: 03785966.7
(22) Date of filing: 17.12.2003
(51) Int. Cl.: C12Q 1/68, A61K 48/00

(54) **NOR-1 (NEURON-DERIVED ORPHAN RECEPTOR-1), NOVEL DIAGNOSTIC AND THERAPEUTIC TARGET FOR CARDIOVASCULAR DISEASES**

(30) Priority: 17.12.2002 ES 200202901
(71) Applicant: CONSEJO SUPERIOR DE INVESTIGACIONES CIENTIFICAS, 28006 Madrid (ES)
(72) Inventor: Badimon Maestro, Lina, Ctr Invest Cardiovascular, 08025 Barcelona (ES); Martinez Gonzalez, José, 08025 Barcelona (ES)
(74) Representative: Ungria Lopez, Javier
(86) International application number: PCT/ES2003/000644
(87) International publication number: WO 2004/054428

(57) **Abstract**

The invention relates to NOR-1 as a novel target in humans for diagnosing and following-up cardiovascular diseases associated with the activation of NOR-1 or for monitoring, following-up or assessing the effectiveness of pharmacological treatments for said cardiovascular diseases. The invention also relates to NOR-1 as a therapeutic target which can be used to identify novel therapeutic compounds for the treatment of the aforementioned diseases and the design of novel gene therapy protocols.

## Description

### SECTOR OF THE ART

Biomedicine. Novel therapeutic and diagnostic targets for diseases and identification of novel pharmaceutical compounds which regulate the activity of said therapeutic targets. Cardiovascular diseases such as arteriosclerosis.

### STATE OF THE ART

In order to understand the molecular mechanisms involved in the activation and dedifferentiation of vascular smooth muscle cells (VSMC), a detailed mapping is needed of the cascades of transcription factors induced by atherogenic stimuli. These master genes could be targets for new strategies, both therapeutic and diagnostic. Recently, different nuclear receptors, among which can be highlighted Peroxisome Proliferator-Activated Receptors (PPARs) (Marx N, Schonbeck V, Lazar MA, Libby P, Plutzky J. Peroxisome proliferator-activated receptor gamma activators inhibit gene expression and migration in human vascular smooth muscle cells. *Circ Res.* 1998; 83: 1097-1103; Staels B, Koening W, Habib A, Merval R, Lebret M, Torra IP, Delerive P, Faadel A, Chinetti G, Fruchart JC, Najib J, Maclouf J, Tedgui A. Activation of human aortic smooth-muscle cells is inhibited by PPARalpha but not by PPARgamma activator. *Nature* 1998; 393: 790-793), retinoic receptors, both Retinoid X Receptor (RXR) and Retinoic Acid Receptor (RAR) and Retinoid-Related Orphan Receptor (ROR) have been identified in the activation/proliferation of VSMC and consequently implicated in the atherogenic process (Miano JM, Topouzis S, Majesky MW, Olson EN. Retinoid expression and all-trans retinoic acid-mediated growth inhibition in vascular smooth muscle cells. *Circulation.* 1996; 93: 1886-1895; Neuville P, Yan Z, Gidlof A, Pepper MS, Hansson GK, Gabbiani G, Sirsjo A. Retinoic acid regulates arterial smooth muscle cell proliferation and phenotypic features *in vivo* and *in vitro* though an RARalpha-dependent signalling pathway. *Arterioscler Thronb Vasc Biol.* 1999; 19: 1430-1436; Haxsen V, Adam-Stitah S, Ritz E, Wagner J. Retinoids inhibit the actions of angiotensin II on vascular smooth muscle cells. *Circ Res.* 2001; 88: 636-644). Nuclear receptors are a large family of ligand-activated transcription factors, which regulate complex gene programs having great importance in virtually all processes of development and physiology in adult age (Mangelsdorf DJ, Thummel C, Beato M, Herrlich P, Schütz, Umesono K, Blumberg B, Kastner P, Mark M, Chambon P, Evans RM. The nuclear receptor superfamily: the second decade. *Cell.* 1995; 83: 835-839; Giguère V. Orphan nuclear receptors: from gene to function. *Endocr Rev* 1999; 20: 689-725). Also, ligands are currently known for only half the known nuclear receptors, the remaining receptors are known as orphan receptors and especially constitute an area for research and development (R&D).

NOR-1, together with Nur77 and Nurr1, form the NGFI-B subfamily within the superfamily of nuclear receptors (Mangelsdorf DJ, Thummel C, Beato M, Herrlich P, Schütz, Umesono K, Blumberg B, Kastner P, Mark M, Chambon P, Evans RM. The nuclear receptor superfamily: the second decade. *Cell.* 1995; 83: 835-839; Giguère V. Orphan nuclear receptors: from gene to function. *Endocr Rev* 1999; 20: 689-725). These genes have been involved in neuoroendocrine regulation, neuronal differentiation, hepatic regeneration, cell apoptosis and response to mitogenic stimuli in different cell types (Maruyama K, Tsukada t, Ohkura N, Bandoh S, Hosono T, Yamaguchi K. The NGFI-B subfamily of the nuclear receptor superfamily. *Int J Oncol.* 1998; 12: 1237-1243) Finally, it can be expected that new knowledge on the complete functional activity of these transcription factors can permit the development of new diagnostic and treatment strategies for diseases caused by alteration of those potential targets.

### DESCRIPTION

### Brief description

An object of the present invention consists of an identification procedure of NOR-1 and/or Nu77 and/or Nurr1 in biological samples of mammals, preferably humans, for diagnosing and following-up cardiovascular diseases associated with the activation of NOR-1 or for monitoring, following-up or assessing the effectiveness of pharmacological treatments for said cardiovascular diseases, characterised in that it comprises the following parts:
a) Obtaining of the biological sample by means of blood extraction or intravascular surgery (atherectomy or similar), and
b) Identification of NOR-1 and/or Nur77 and/or Nur1 in that biological sample, where a positive or incremented result with respect to a base level considered physiological will indicate the existence of a cardiovascular disease associated with activation of NOR-1 or where the comparison of the levels of NOR-1 determined over time would indicate the evolution of the cardiovascular disease or whether, for example, the treatment administered for that disease is showing any therapeutic effect.

A further object of the present invention consists of an identification procedure of potential modulator compounds of NOR-1 activity and, therefore, potential therapeutic compounds of cardiovascular diseases associated with the activation of NOR-1 and/or Nur77 and/or Nur1, characterised by the following stages:
a) placing of said compound in contact in a biological environment where there exists the functionally active protein NOR-1 and/or Nur77 and/or Nur1,
b) determination of the expression levels or activity levels of the protein of NOR-1 and/or Nur77 and/or Nur1, and
c) selection of said compound as modulator compound of NOR-1 and/or Nur77 and/or Nur1 if the inhibition or boosting of the effects of NOR-1 is noted in the presence of the compound.

A particular object of the present invention consists of an identification procedure of potential modulator compounds of NOR-1 and/or Nur77 and/or Nur1 activity where the "biological environment" is for example a VSMC culture of mammals, preferably human or porcine, where in the presence or otherwise of NOR-1 inductor mitogens - serum, thrombin, PDGF and EGF, among others - the potential compound to be assessed is added and a determination is then made of: mRNA levels of NOR-1 and/or Nur77 and/or Nur1 (by means of RT-PCR, Northern blot, *in situ* RT-PCR or *in situ* hybridation, among other methods) or the NOR-1 and/or Nur77 and/or Nur1 activity [by means of the capacity of these transcription factors to bind themselves to specific DNA sequences in Electrophoretic Mobility Shift Assays (EMSA)] as has been done in the present invention, or determining the capacity of NOR-1 to form heterodimers with other members of the NGFI-B family (Nur77 and Nurr1) thereby modulating the transactivation of target genes; or by means of determination of the protein levels (by means of the techniques mentioned above).

Another particular object of the present invention consists of an identification procedure of potential modulator compounds of NOR-1 and/or Nur77 and/or Nur1 activity where the "biological environment" is for example an animal model, preferably porcine, which is subjected to a percutaneous transluminal coronary angioplasty (PTCA) operation, it is administered with the potential compound to assess and then a determination is made of the NOR-1 levels of the lesions caused by means of techniques such as RT-PCR, Northern blot, *in situ* RT-PCR or Western blot (see example 2.3 of the present invention) among others.

So, a further object of the present invention consists of a NOR-1 inhibitor or booster compound (which will modify its expression or its activity as a transcription factor individually or cooperatively with the other members of the NGFI-B family) identified according to a procedure described above. Equally, a further object consists of a pharmaceutical composition characterised in that it comprises a therapeutically effective quantity of a NOR-1 inhibitor or booster compound identified by the procedure of the present invention.

A further object of the present invention consists of the use of a pharmaceutical composition described above in the preparation of a medicine for the treatment of cardiovascular diseases associated with activation of NOR-1 in mammals, preferably humans.

A further object of the present invention is a gene therapy procedure for the treatment and prevention in mammals, preferably humans, of cardiovascular diseases associated with activation of NOR-1, which would permit the modification of NOR-1 expression in cells involved in the aetiopathogeny of those diseases, for example by means of the expression of antisense oligonucleotides of NOR-1 (ODNs) using vectors or carriers permitting the entrance of those ODNs into those cells. A particular object of the present invention consists of antisense/against NOR-1 ODNs described in this invention: AS1-NOR-1 (SEQ ID NO6) and AS2-NOR-1 (SEQ ID NO 7), along with any other that might be developed with the knowledge existing in the state of the art, and which block the expression of NOR-1 (Nabel EG, Nabel GJ, Gene Transfer. In: *Atherosclerosis and Coronary Artery Disease.* edited by V. Fuster, R Ross and EJ Topol. Lippincott-Raven Publisher, Philadelphia, 190996). Said strategies would also include those directed towards modifying the function of the NOR-1 protein by means of over-expression of a defective (dominant negatives) NOR-1 protein (or of another member of the NGFI-B family) which compete with the native proteins and reduce their activity.

### Detailed description

In this study, use was made of the mRNA-DD analysis system in a search for mitogen-induced genes in VSMC in the porcine model, a model which, due to its great similarity to the human one, is commonly accepted in the study of VSMC differentiation and in arteriosclerosis (Badimon L, Atherosclerosis and thrombosis: lessons from animal models. *Thromb Haemost.* 2001; 86, 356-365). We have identified NOR-1 as a rapid induction gene in VSMC. NOR-1 was temporarily induced by means of angioplasty in coronary arteries of pig and was overexpressed in primary atherosclerotic lesions in humans. Induction of the other members of the NGFI-B family was also detected in human atherosclerotic lesions (Nur77) and in porcine coronary arteries subjected to balloon angioplasty (Nurr1). Antisense ODNs against NOR-1 inhibited VSMC proliferation and tissue repair induced by means of mechanical damage.

Starting from porcine SMC, two isoforms of NOR-1 were cloned. NOR-1 is a rapid induction gene which was intensely induced in VSMC by mitogenic stimuli which signal by means of dependent pathways of protein kinase receptors (PDGF and EGF) and of receptors coupled to G proteins (thrombin). On the contrary, other agents, such as cytokines, which induce NOR-1 in gingival fibroblasts (Borghaei RC, Sinai ES, Mochan E, Pease EA. Induction of mitogen-inducible nuclear orphan receptor by interleukin 1 in human synovial and gingival fibroblasts. *Biochem Biophys Res Common.* 1998; 251: 334-338) or angiotensin-II, which induces NOR-1 in fasciculate cells (Fernández PM, Brunel F, Jiménez MA. Saez JM, Cereghini S, Zakin MM. Nuclear receptors Nor1 and NGFI-B/Nur77 play similar, albeit distinct, roles in hypothalamo-pituitary-adrenal axis. *Endocrinology.* 2000; 141: 2392-2400), did not induce NOR-1 in VSMC. Increase in the intracellular calcium concentration along with activation of PKC and the MAPKs pathway, pathways activated in cell migration and proliferation processes, are involved in NOR-1 induction in VSMC. In this sense, mitogens using more than one pathway (for example, serum) induced NOR-1 to a greater degree, and the association of PDGF or thrombin with IGF-1 increased the effect individually produced by each one of them. Therefore, NOR-1 could play a key role integrating different signalling pathways in VSMC, in processes such as thrombosis which produce the activation/degranulation of the platelets (which release PDGF and EGF) and generation of thrombin. If fact, NOR-1 was also induced by these agents in other cells involved in the atherosclerotic process, such as endothelial cells and monocytes/macrophages [Fuster V, Badimon L, Badimon JJ, Chesebro JH. The pathogenesis of coronary artery disease and the acute coronary syndromes. New Eng J Med 326. Part I (242-250) and Part (310-318)]. Also, growing concentrations of low density lipoproteins (LDLs) induced NOR-1 both in VSMC and in endothelial cells, while growing concentrations of HDL only induced NOR-1 in VSMC. NOR-1 modulation by lipoproteins suggests that this gene can be a potential indicator of processes associated with an increase in the circulating levels of LDL, which in fact, constitutes one of the best characterised atheroscleritic risk factors having an impact on the physiology of both vascular cells (VSMC and endothelial cells) and circulating cells (particularly monocytes) (Navad M, Fogelman AM, Berliner JA, Terroto MC, Demer LL, Franks JS, Watson AD, Edwards PA, Lusis AJ. Pathogenesis of atherosclerosis. Am J Cardiol 1995; 76: 18C-23C; Lusis AJ. Atherosclerosis Nature 2000; 407: 233-241).

Transfection experiments indicate a key role for CRE elements present in the NOR-1 promoter in its induction by mitogenic stimuli. In fact, CREB activation is a point of convergence both of PKC dependent pathways and of calcium mobilisation (Mayr B, Montminy M, Transcriptional regulation by the phosphorylation-dependent factor CREB. *Nat Rev Mol Cell Bio* 2001; 2: 599-609), the two main pathways involved in NOR-1 induction. Serum induced the phosphorylation of CREB, though no changes were observed in the binding of CREB, according to the capacity of CREB to bind itself constitutively to its response element (Mayr B, Montminy M, Transcriptional regulation by the phosphorylation-dependent factor CREB. *Nat Rev Mol Cell Bio* 2001; 2: 599-609).

Although NOR-1 is temporarily induced by mitogens in cultured cells, significant expression of NOR-1 was detected in various adult tissues, particularly in the myocardium and in striated muscle, both of pig and of humans (data not shown), which suggests that in those tissues NOR-1 could regulate genes involved in key functions in them. The simultaneous expression of the genes of the NGFI-B family in certain tissues and cells, the great homology of their amino-acid sequences and the capacity of the three receptors to bind to a specific response element (NBRE) (Wilson TE, Fahmer TJ, Johnston M, Milbrandt J. Identification of the cDNA binding site for NGFI-B by genetic selection in yeast. *Science.* 1991; 256: 1296-1299) can explain the functional redundancy observed in certain systems (Cheng LEC, Chan FKM, Cado D, Winoto A. Functional redundancy of the Nur77 and Nor-1 orphan steroid receptors in T-cell apoptosis. *EMBO J.* 1997; 16: 1865-1875). Nevertheless, we observed different expression patterns in adult tissues and on the vascular wall under pathological conditions. In fact, although both NOR-1 expression and that of Nur77 are increased in human primary atherosclerotic lesions in patients with coronary artery disease (CAD) or aortic aneurysm, the revascularisation procedures that damage the vascular wall and induce the proliferation of VSMC activated the expression of NOR-1 and Nurr1 but not that of Nur77 (data not shown). The significance of the increased expression of Nur77 and Nurr1 in these samples is not currently known. Nevertheless, Nur77 and Nurr1 are induced concomitantly with NOR-1 (in the situations mentioned), they bind to a single response element and they can form heterodimers with each other (Maira M, Martens C, Philips A, Drouin J. Heterodimerisation between members of the Nur subfamily of orphan nuclear receptors as a novel mechanism for gene activation. Mol Cell Biol 1999 ; 19: 7549-7557) or with RXR (Mangelsdorf DJ, Evans RM. The RXR heterodimers and orphan receptors. *Cell.* 1995; 83: 841-850), nuclear factor which modulates the VSMC phenotype (as discussed more fully in the following paragraph), they could thus positively or negatively modulate NOR-1 activity in vascular cells or in blood cells. Therefore, both Nur77 and Nurr1 would be directly or indirectly involved in pathologies in which NOR-1 induction is detected.

The genes of the NGBI-B family have been involved in different cell functions, among them cell proliferation and differentiation and apoptosis (Maruyama K, Tsukada t, Ohkura N, Bandoh S, Hosono T, Yamaguchi K. The NGFI-B subfamily of the nuclear receptor superfamily. *Int J Oncol.* 1998; 12: 1237-1243). It must be borne in mind that in the same way these "unspecific" roles have been attributed to other early response genes such as *c-fos* and *c-myc* (Rothman A, Wolner B, Button D, Taylor P. Immediate-early gene expression in response to hypertrophic and proliferative stimuli in pulmonary arterial smooth muscle cells. *J Biol Chem.* 1994; 269: 639-6404; Macdonald K, Bennet MR. Cdc25A is necessary but not sufficient for optimal c-myc induced apoptosis and cell proliferation of vascular smooth muscle cells. *Circ Res.* 1999; 84; 820-830). In this sense, it seems plausible that in a particular physiological context the specific function of NOR-1 would depend on concomitant changes in the expression levels of other genes. In fact, the relation between eukaryote transcription factors, particularly the paradigmatic heterodimerisation between nuclear receptors (Maira M, Martens C, Philips A, Drouin J. Heterodimerisation between members of the Nur subfamily of orphan nuclear receptors as a novel mechanism for gene activation. Mol Cell Biol 1999 ; 19: 7549-7557), increases the complexity of the molecular "networks" operating in vascular cells. Nur77 and Nurr1 (but not NOR-1) can form heterodimers with RXR (Mangelsdorf DJ, Evans RM. The RXR heterodimers and orphan receptors. *Cell.* 1995 ; 83: 841-850), permitting the latter to activate the vascular genes in a ligand dependent way via response elements to retinoic acid. Given that RXR has been directly involved in the modulation of VSMCs phenotype and in atherogenesis (Haxsen V, Adam-Stitah S, Ritz E, Wagner J. Retinoids inhibit the actions of angiotensin II on vascular smooth muscle cells. *Circ Res.* 2001; 88: 636-644; Claudel T, Leibowitz MD, Fievet C, Tailleux A, Wagner B, Repa JJ, Torpier G, Lobaccaro JM, Paterniti JR, Mangelsdorf DJ, Heyman RA, Auwerx J. Reduction of atherosclerosis in apolipoprotein E knockout mice by activation on the retinoid X receptor. *Proc Natl Acad Sci USA.* 2001; 27: 2610-2615), and is the common partner of other nuclear receptors activated in vascular cells such as PPARs or RAR (Marx N, Schonbeck V, Lazar MA, Libby P, Plutzky J. Peroxisome proliferator-activated receptor gamma activators inhibit gene expression and migration in human vascular smooth muscle cells. *Circ Res.* 1998; 83: 1097-1103; Haxsen V, Adam-Stitah S, Ritz E, Wagner J. Retinoids inhibit the actions of angiotensin II on vascular smooth muscle cells. *Circ Res.* 2001; 88: 637-644; Benson S, Padmanabhan S, Kurtz TW, Pershadsingh HA. Ligands for the peroxisome proliferator-activated receptor-gamma and the retinoid X receptor-alpha exert synergistic antiproliferative effects on human coronary artery smooth muscle cells. *Mol Cell Bio Res Commun.* 2000; 3: 159-164), a potential "competition" of these heterodimer pairs by RXR should not be discarded. This interference/synergy relation between nuclear receptors, among themselves or with other transcription factors can be important in regulating the function of vascular cells (Benson S, Padmanabhan S, Kurtz TW, Pershadsingh HA. Ligands for the peroxisome proliferator-activated receptor-gamma and the retinoid X receptor-alpha exert synergistic antiproliferative effects on human coronary artery smooth muscle cells. *Mol Cell Bio Res Commun* 2000; 3: 159-164).

NOR-1 expression pattern is in agreement with the participation of this transcription factor in the underlying molecular mechanisms in both spontaneous and "accelerated" arteriosclerosis. The nature of the stimuli leading to an increase in NOR-1 expression, both *in vivo* and *in vitro,* and the intracellular pathways involved in that induction firmly support a role for NOR-1 in the proliferation of VSMCs. In order to back up this hypothesis we analyse the effect of two antisense ODNs directed against NOR-1. These ODNs significantly inhibited the proliferation of human coronary SMC (they reduced the de *novo* synthesis of DNA, cell cycle progression and lesion repair by VSMCs). The effectiveness of the antisense ODNs blocking these processes was similar to that produced by ODNs directed against the well-characterised proto-oncogene *c-fos,* which as NOR-1 is temporarily induced in VSMC and in porcine coronary arteries following PTCA (Martínez-González J, Viñals M, Vidal F, Llorente-Cortés V, Badimon L, Mevalonate deprivation impairs IGF-I/Insulin signalling in human vascular smooth muscle cells. *Atherosclerosis* 1997; 135: 213-223; Rothman A, Wolner B, Button D, Taylor P. Immediate-early gene expression in response to hypertrophic and proliferative stimuli in pulmonary arterial smooth muscle cells. *J Biol Chem.* 1994; 269: 639-6404;, Badimon L, Alfon J, Royo T, Berrozpe M, Martínez-González J, Vidal F, Chesebro JH, Fuster V, Badimon JJ., Cell biology of restenosis post-angioplasty. *Z Kardiol.* 1995; 84: 145-149). We selected *c-fos* as the comparison pattern because a proportionality relation has been established between the degree of expression of *c-fos* and VSMC proliferation both *in vivo* (Indolfi C, Esposito G, Di Lorenzo E, Rapacciuolo A, Feliciello A, Porcellini A, Avvedimento VE, Condorelli M, Chiariello M. Smooth muscle cell proliferation is proportional to the degree of balloon injury in a rat model of angioplasty. *Circulation.* 1995; 92: 1230-1235) and *in vitro* (Martínez-González J, Viñals M., Vidal F, Llorente-Cortés V, Badimon L, Mevalonate deprivation impairs IGF-I/Insulin signalling in human vascular smooth muscle cells. *Atherosclerosis* 1997; 135: 213-223), and ODNs against *c-fos* have been successfully used to block cell proliferation in different systems including VSMC (Leon T, Vazquez E, Sanz C, Vega JA, Mato JM, Giraldez F, Represa J, Varela-Nieto I., Insulin-like growth factor-I regulates cell proliferation in the developing inner ear, activating glycosyl-phosphatidylinositol hydrolysis and Fos expression. *Endocrinology.* 1995; 136: 3494-3503; Suggs W, Olsen SC, Mandani D, Patel S, Eith FJ. Antisense oligonucleotides to *c-fos* and *c-jun* inhibit intimal thickening in a rat vein graft model. *Surgery.* 1999; 126: 443-449). Our results firmly suggest that NOR-1 plays a role in VSMCs proliferation, which is in agreement with the evidence obtained by other authors who attribute a role to this orphan nuclear receptor in cell proliferation in different systems. In fact, among the NGFI-B gene family, only the oncogenic conversion of NOR-1 has been described (Labelle Y, Zucman J, Stenman G, Kindblom LC, Knight J, Turc-Carel C, Dockhom-Dwomiczak B, Mandahl N, Desmaze C, Peter M. Aurias A, Delattre O, Thomas G. Oncogenic conversion of a novel orphan nuclear receptor by chromosome translocation. *Hum Mol Genet.* 1995; 4: 2219-2226).

To summarise, the present invention is based on the fact that the inventors have observed that NOR-1 transcription factor is a potential modulating factor of vascular cell function (VSMCs and endothelial cells) and monocytes/macrophages, and therefore a new diagnostic target for cardiovascular pathologies and a new target in future therapeutic strategies (pharmacological or gene-therapy based).

Thus, an object of the present invention constitutes an identification procedure of NOR-1 and/or Nu77 and/or Nurr1 in biological samples of mammals, preferably humans, for diagnosing and following-up cardiovascular diseases associated with NOR-1 activation or for monitoring, following-up or assessing the effectiveness of pharmacological treatments for said cardiovascular diseases, characterised in that it comprises the following parts:
a) Obtaining the biological sample by means of blood extraction or intravascular surgery (atherectomy or similar), and
b) Identification of NOR-1 and/or Nur77 and/or Nur1 in said biological sample, where a positive or incremented result with respect to a base level considered physiological will indicate the existence of a cardiovascular disease associated with activation of NOR-1 or where the comparison of NOR-1 levels determined over time would indicate the evolution of the cardiovascular disease or whether, for example, the treatment administered for said disease is showing any therapeutic effect.

As used in the present invention, the term "identification of NOR-1" refers to the determination of human NOR-1 expression levels, in any of its two alpha and beta isoforms, and/or Nur77 and/or Nurr1, be it its messenger RNA or the corresponding proteins - by means of any quantitative, semi-quantitative or qualitative method - in a biological sample of a potential patient of said cardiovascular disease.

The term "biological sample" as used in the present invention refers to body fluids such as blood (especially to its constituent cells), to vascular cells and to samples of peripheral tissues affected by cardiovascular diseases or by processes that can act as coadjuvants in the development of atherosclerotic lesions and eventually as markers of the process (for example, biopsies of gums), obtained by means of blood extraction, intravascular surgery (atherectomy or similar) among others.

As used in the present invention, the term "quantitative, semi-quantitative or qualitative method" refers to methods used in the field of molecular biology for the determination of mRNA levels such as, among others, RT-PCR, Northern blot (Sambroock J, Fritsch EF, Maniatis T. Extraction purification analysis of messenger RNA from eukaryotic cells. In: Molecular Cloning (book 1). Could Spring Harbor Laboratory Press, 1989: 7.39), "DNA arrays" or biochips of DNA or oligonucleotides (Hitunen MO, Niemi M, Ylä-Herttuala S. Functional genomics and DNA array techniques in atherosclerosis research. Curr Opin Lipidol. 1999; 10: 515-519); *in situ* hybridation or in situ RT-PCR (Herrington GS, McGee JO. Principles and basic methodology of DNA/RNA detection by *in situ* hybridation. In: Herrington GS, McGee JO. Eds Diagnostic molecular pathology: A practical approach,. IRL press, 1992; 1: 69-102); or of protein levels by means of polyclonal or monoclonal antibodies which recognise NOR-1, such as, among others, Western blot (Sambroock J, Fritsch EF, Maniatis T. Detection and analysis of proteins expressed from cloned genes. In: Molecular Cloning (book 3). Cold Spring Harbor Laboratory Press, 1989: 18.60), immunocytochemistry and immunohistochemistry (Robinson G, Ellis IO, MacLennan A,. Immunochemistry. In: Bandcroft JD, Stevens A. ed., Theory and practice of histological techniques. Churchill Livingstone, 1990: 413-436), EIA or ELISA techniques (Pradellas P., et al. Enzyme immunoasasays of eicosanoids using acetylcholinesterase as label: an alternative to radiommunoassays. Anal Chem 1985; 57: 1170-1173), protein biochips (Huang RP. Detection of multiple proteins in an antibody-based protein microarray system. Journal of Immunological Methods 225 (2001): 1-13; **http://www.functionalgenomics.org.uk/sections/resources/p roteinarrays.htm;** Kodadek T. Protein microarrays: prospects and problems. Chemistry and Biology 8/2 (2001): 105-115). Currently at the development phase by the inventors of the present invention is the obtention of antibodies against a fragment of NOR-1 protein sequence generated by expression in a plasmidic prokaryote vector, by means of techniques widely known by any expert in the sector of the art of this invention (Sambroock J, Fritsch EF, Maniatis T. Expression of cloned genes in *Escherichia coli.* In: Molecular Cloning (book 3). Cold Spring Harbor Laboratory Press, 1989).

A further object of the present invention consists of an identification procedure of potential modulatory compounds of NOR-1 activity and, therefore, potential therapeutic compounds for cardiovascular diseases associated with NOR-1 and/or Nur77 and/or Nur1 activation, characterised by the following stages:
a) placing said compound in contact with a biological environment where functionally active NOR-1 and/or Nur77 and/or Nur1 proteins exist,
b) determination of the expression levels or activity levels of NOR-1 and/or Nur77 and/or Nur1 proteins, and
c) selection of said compound as modulator compound of NOR-1 and/or Nur77 and/or Nur1 if the inhibition or boosting of NOR-1 effects is noted in the presence of the compound.

A particular object of the present invention consists of an identification procedure of potential modulatory compounds of NOR-1 and/or Nur77 and/or Nur1 activity where the "biological environment" is for example a VSMC culture of mammals, preferably human or porcine, where in the presence or otherwise of NOR-1 inductor mitogens - serum, thrombin, PDGF and EGF, among others - the potential compound to be assessed is added and a determination is then made of: mRNA levels of NOR-1 and/or Nur77 and/or Nur1 (by means of RT-PCR, Northern blot, in situ RT-PCR or in situ hybridation, among other methods) or NOR-1 and/or Nur77 and/or Nur1 activity [by means of the capacity of these transcription factors to bind themselves to specific DNA sequences in Electrophoretic Mobility Shift Assays (EMSA)] as has been done in the present invention, or determining the capacity of NOR-1 to form heterodimers with other members of the NGFI-B family (Nur77 and Nurr1) thereby modulating the transactivation of target genes; or by means of determining protein levels (using the techniques mentioned above).

Another particular object of the present invention consists of an identification procedure for potential modulatory compounds of NOR-1 and/or Nur77 and/or Nur1 activity where the "biological environment" is for example an animal model, preferably porcine, which is subjected to a percutaneous transluminal coronary angioplasty (PTCA) operation, it is administered with the potential compound to assess, and then a determination is made of NOR-1 levels of the lesions caused by means of techniques such as RT-PCR, Northern blot, *in situ* RT-PCR or Western blot (see example 2.3 of the present invention) among others.

Thus, a further object of the present invention consists of a NOR-1 inhibitor or booster compound (which will modify its expression or its activity as a transcription factor individually or cooperatively with the other members of the NGFI-B family) identified according to a procedure described above. Equally, a further object consists of a pharmaceutical composition characterised in that it comprises a therapeutically effective quantity of a NOR-1 inhibitor or booster compound identified by the procedure of the present invention.

A further object of the present invention consists in the use of a pharmaceutical composition described above in the preparation of a medicine for the treatment of cardiovascular diseases associated with activation of NOR-1 in mammals, preferably humans.

A further object of the present invention is a gene therapy procedure for the treatment and prevention in mammals, preferably humans, of cardiovascular diseases associated with activation of NOR-1, which would permit the modification of NOR-1 expression in cells involved in the aetiopathogeny of those diseases, for example by means of the expression of antisense oligonucleotides of NOR-1 (ODNs) using vectors or carriers that permit the entrance of those ODNs into said cells. A particular object of the present invention consists of antisense ODNs/against NOR-1 described in this invention: AS1-NOR-1 (SEQ ID N06) and AS2-NOR-1 (SEQ ID NO 7), along with any other that might be developed with the knowledge existing in the state of the art, and that block NOR-1 expression (Nabel EG, Nabel GJ, Gene Transfer. In: *Atherosclerosis and Coronary Artery Disease.* edited by V. Fuster, R Ross and EJ Topol. Lippincott-Raven Publisher, Philadelphia, 1996). Said strategies would also include those directed towards modifying the function of the NOR-2 protein by means of over-expression of a defective (dominant negatives) NOR-1 protein (or of another member of the NGFI-B family) that competes with the native proteins and reduce their activity.

As used in the present invention, the term "cardiovascular diseases associated with activation of NOR-1" refers to cardiovascular diseases which are associated with increased levels of NOR-1, due to an inductor causal relation or simply by an association, which belongs to, among others, the following group: atherothrombosis (Woof N, Davies MJ, Interrelationship between atherosclerosis and thrombosis. In: *Thrombosis in cardiovascular Disorders.* Edited by V Fuster and M Verstraete. WB Sanders Company. Harcourt Brace Jovanovich, Inc. Philadelphia 1992), "accelerated" arteriosclerosis (post-vascularisation restenosis) (Nikol S, Huehns TY, Höfling B. Molecular biology and post-angioplasty restenosis. Atherosclerosis 1996; 123: 17-23), spontaneous arteriosclerosis in its different manifestations [coronary artery disease (CAD), aortic disease, cerebrovascular disease and peripheral arteriopathies] (Ross R. The pathogenesis of atherosclerosis. Nature 1993; 362: 801-809; Lusis AJ. Atherosclerosis Nature 2000; 407: 233-241), aneurysms and vascular remodelling/regeneration (Ouriel K. Endosvascular therapies: an update on aortic aneurysm repair and carotid endarterectomy. J Am Coll Surg. 2002; 195: 549-552) and in general cardiovascular diseases associated with de-differentiation (Owens GK. Role of alterations in the differentiated state of vascular smooth muscle cells in atherogenesis. In: *Atherosclerosis and Coronary Artery Disease.* Edited by V Fuster, R Ross and EJ Topol. Lippincott-Raven Publisher, Philadelphia, 1996), migration and proliferation (Libby P. Molecular bases of the acute coronary syndromes. Circulation 1995; 91: 2844-2850) or apoptosis of vascular cells (Best PJM et al. Apoptosis: basic concepts and implications in coronary artery disease. Arterioscler Throm Biol 1999 ; 19: 14-22) or which cause endothelial dysfunction such as hypercholesterolemia (John S, Schmieder RE. Impaired endothelial dysfunction in arterial hypertension and hypercholesterolemia: potential mechanisms and differences.

### DESCRIPTION OF THE FIGURES

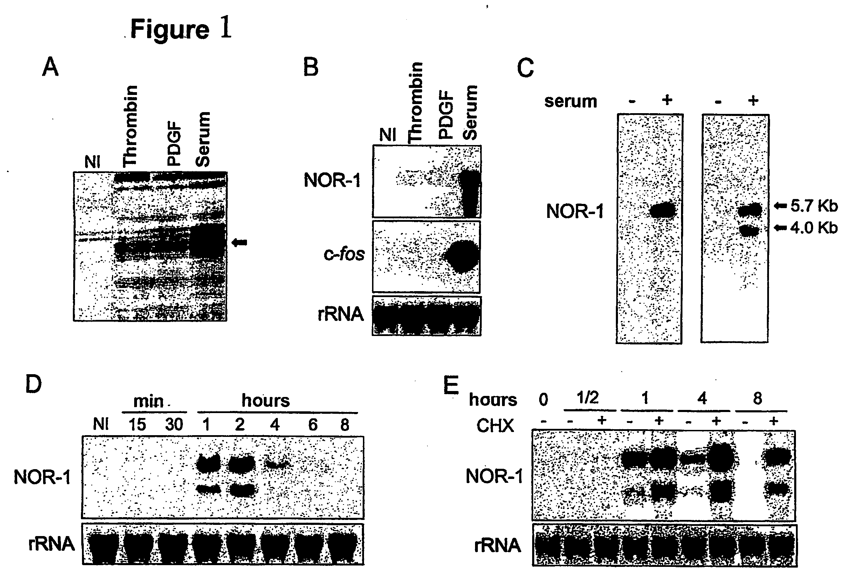

**Figure 1.- Identification of NOR-1 by means of mRNA-DD.** Representative mRNA-DD analysis showing the induction of band 2 produced by 10% human serum, 10 U/mL of thrombin and 5 nmol/L of PDGF (after 1 h of stimulation). B, *Northern blot* analysis of quiescent versus stimulated porcine coronary artery SMC using differentially regulated cDNA (band 2) or *c*-*fos* as probes. rRNA ribosomal RNA. C. *Northern blot* of quiescent *versus* stimulated porcine coronary artery SMC (1 h with serum) using the cDNA from band 2 (*left*) or pNOR-1α (*right*) as probes. D. NOR-1 induction kinetics by serum in human coronary SMC. E, mRNA levels of NOR-1 in human coronary SMC stimulated with serum in presence or absence of cycloheximide (CHX) for different times. Nl, non-induced cells.

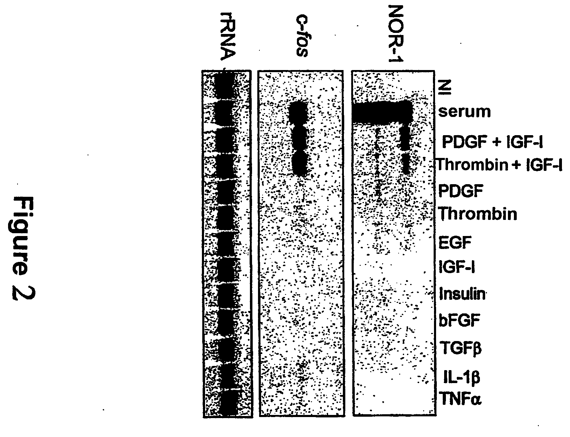

**Figure 2.- NOR-1 Expression in human VSMC**. mRNA levels in human coronary VSMC stimulated with growth factors and cytokines for 1 h. A, A representative *Northern blot* is shown (n=3) [rRNA, ribosomal RNA; bFGF, basic fibroblast growth factor; TGFβ, transforming growth factor beta; IL-1β, interleucine-1 beta; TNF-α, tumour necrosis factor alpha]. Nl, non-induced cells.

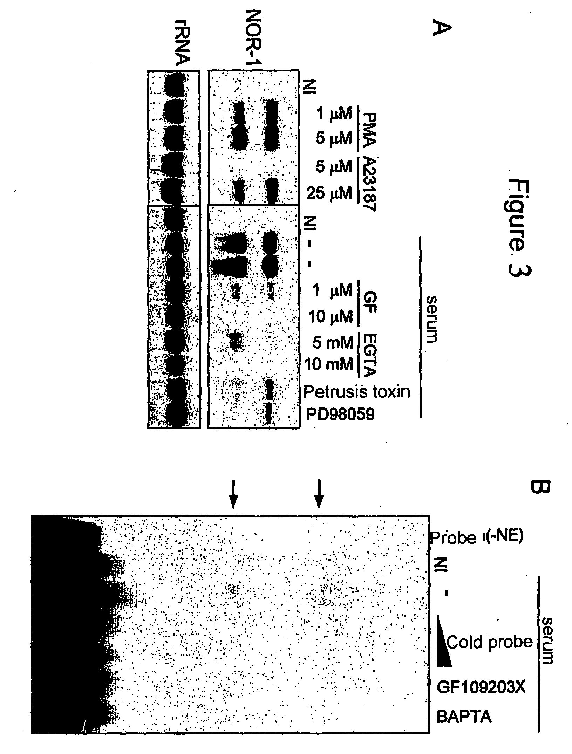

**Figure 3.- Signalling pathways involved in NOR-1 induction.** A, *Northern blot* showing NOR-1 expression in human coronary VSMC stimulated with PMA or with calcium ionophore A23187 for 1 h, and the effect of inhibitors of different signalling pathways. A representative experiment is shown (n=3). [GF,GF-109203X]. B, EMSA showing the increase in the binding of nuclear extracts from cells treated with serum to a probe containing a consensus NBRE element and the effect of GF-109203X and BAPTA (a calcium chelate). The competition of the binding due to an excess (5 and 50 times) of cold probe is shown. The arrows indicate the shift bands. [Probe(-NE), NBE probe without nuclear extracts]. Nl, non-induced cells.

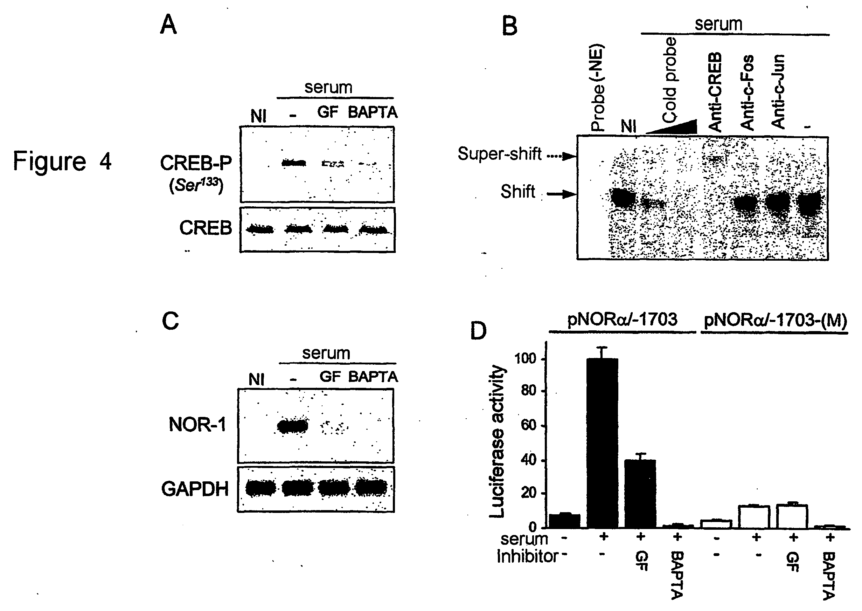

**Figure 4.- Mechanisms involved in NOR-1 induction.** A, Western blot showing the effect of serum (after 10 min) on the phosphorylation of CREB on serine¹³³ (CREB-P) and the effect of inhibitors GF-109203X and BAPTA. Shown in the lower panel are the total levels of CREB. B, EMSA showing the binding capacity of nuclear extracts from human VSMC to the probe Nor/3CRE. The effect of growing quantities of cold probe (5 and 50 times) is shown. The super-shift band produced by anti-CREB antibodies but not by anti-c-Fos or anti-c-Jun antibodies is shown. [Probe(-NE), Nor/CRE probe without nuclear extracts]. C, Effect of 10% serum, GF-109203X and BAPTA on the levels of NOR-1 in NIH-3T3 cells. D. Serum increases the activity of NOR-1 promoter in cells transfected with the native promoter (pNORα/-1703) but not in those transfected with the promoter in which the three CRE elements were mutated [pNORα/-1703-(M)]. The effect produced by the inhibition of the PKC and calcium mobilisation is shown. The luciferase activity was normalised with β-galactosidase activity and was calculated as a percentage of the pNORα/-1703 activity in cells treated with serum. N1, non-induced cells.

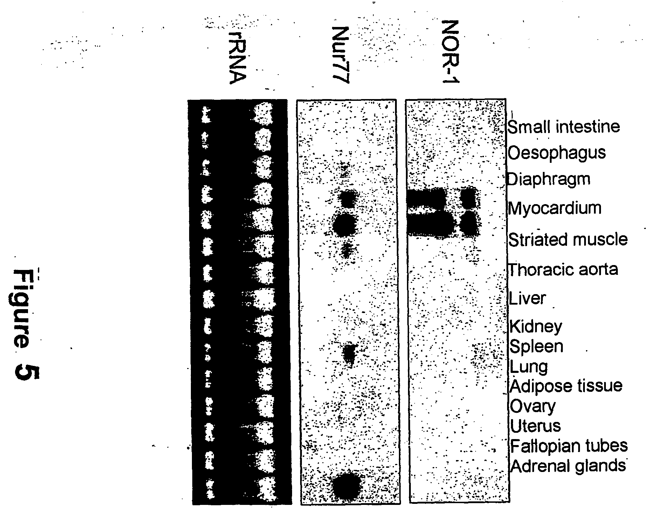

**Figure 5.- NOR-1 expression in porcine tissues.** *Northern blot* showing the mRNA levels of NOR-1 and Nur77 in porcine tissues. A representative experiment is shown (n=3). [rRNA, ribosomal RNA]

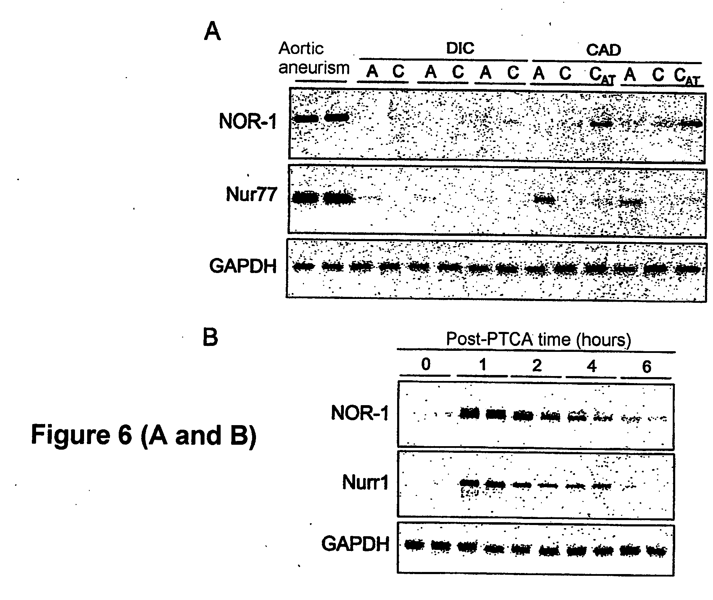

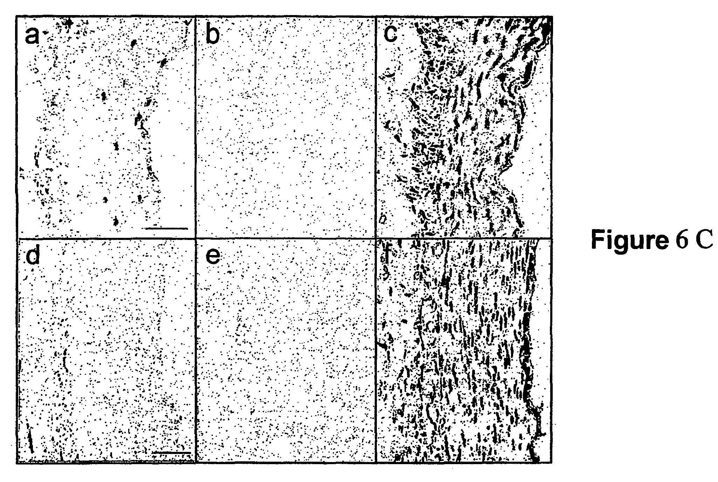

**Figure 6.-** Expression of members of the NGFI-B family in human and porcine arteries **A**, NOR-1 and Nur77 expression in human arteries of patients with aortic aneurysm, dilated idiopathic cardiopathy (DIC) and coronary artery disease (CAD). [A, intima/media aorta; C, intima/media of non-atherosclerotic coronary arteries ; C_{AT} intima/media of atherosclerotic coronary arteries]. **B,** NOR-1 and Nur77 regulation by balloon angioplasty. Representative experiment showing two dilated arteries each time. [PTCA, percutaneous transcutaneous coronary angioplasty]. **C**, *in situ* RT-PCR showing the induction of NOR-1 in SMC of the media following PTCA. Seriate cuts of dilated (a-c) and non-dilated (d-f) arteries; b and e correspond to negative controls of RT-PCR; c and f correspond to haemotoxylin-eosin stainings. Bar = 50 µm.

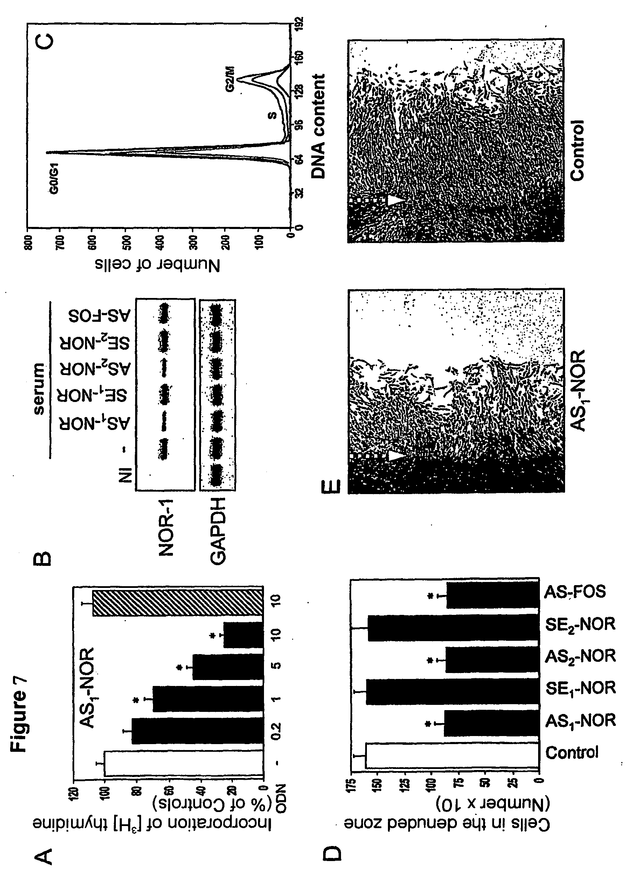

**Figure 7.- Antisense oligonucleotides against NOR-1 inhibit DNA synthesis and tissue repair of human coronary SMC. A,** Quiescent VSMCs were stimulated with 10% human serum for 24 h and a determination of the incorporation of [³H] thymidine was made in the absence (white bars) or presence of growing concentrations (µmol/L) or antisense ODNs (AS₁-NOR, black bars).
   The effect is shown of the corresponding "sense" sequence (SE₁-NOR, 10 µmol/L) (striped bars); (n=4 experiments conducted in triplicate). B, Effect of the oligonucleotides on the mRNA levels of NOR-1 analysed by means of RT-PCR 3 h after the stimulation with serum. [AS-FOS, antisense ODNs against *c-fos*]*.* **C,** Representative profiles of the analysis by means of flow cytometry of quiescent human VSMC (black line), stimulated with serum (red line) and treated with AS₁-NOR (blue line). **D,** Antisense oligonucleotides against NOR-1 inhibit tissue repair after mechanical damage of human coronary VSMC. Confluent/quiescent SMC were scraped and then incubated for 72 h in the absence (Control) or presence of oligonucleotides (10 µmol/L). The histogram shows the number of cells in the denuded zone (n=3 experiments conducted in triplicate). In **E** the representative data corresponding to cells treated with AS₁-NOR and controls is shown. The arrows indicate the margin of the "wound" [ODNs, oligonucleotides; AS- antisense; SE- sense]; (*, p<0.05, *versus* controls or cells treated with the corresponding sense sequence). N1, non-induced cells.

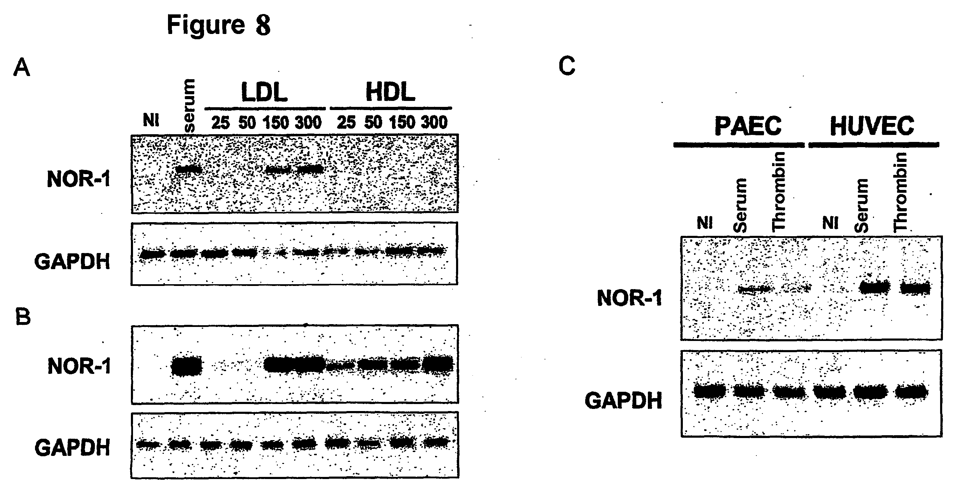

**Figure 8.- NOR-1 induction by lipoproteins and mitogens in human endothelial cells and by lipoproteins in VSMC.** A and B, levels of NOR-1 mRNA in human endothelial cells (HUVEC) (A) and in human coronary VSMC (B) in response to growing concentrations of LDL or HDL (µg/mL). The levels of GAPDH used for normalising the result are shown. As control, the effect of 10% human serum is shown. C, Levels of NOR-1 mRNA of HUVEC and porcine endothelial cells (PAEC) in culture activated with 10% human serum or thrombin (10 U/mL). A representative experiment is shown (n=3) of the levels of mRNA determined by means of RT-PCR. N1, non-induced cells.

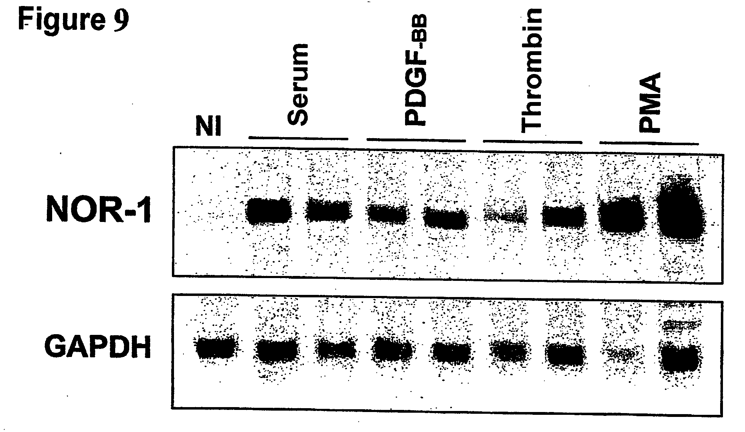

**Figure 9.- NOR-1 induction in monocytes/macrophages.** Levels of NOR-1 mRNA in human monocytes and macrophages in culture activated with 10% human serum, PDGF-BB (5 mmol/L), thrombin (10 U/mL) and a PKC inductor (PMA 5 µmol/L). The levels of GAPDH used for normalising the result are shown. A representative experiment is shown (n=3).

### EXAMPLES OF EMBODIMENT

The following Examples serve to illustrate the invention and must not be considered as limiting its scope.

### Example 1.- The NOR-1 transcription factor is induced in VSMC by mitogens of CREB dependent mechanisms

### - Analysis by means of DD/RT-PCR of VSMC

In order to identify new genes involved in the activation of VSMCs, porcine coronary artery SMCs were stimulated with mitogens such as serum, PDGF or thrombin and the differential gene expression was analysed by means of mRNA-DD analysis. The porcine model is, on account of its great similarity to the human one, a commonly accepted model in the study of VSMC differentiation and in arteriosclerosis (Badimon L, Atherosclerosis and thrombosis: lessons from animal models. *Thromb Haemost.* 2001; 86, 356-365). A total of 23 bands were isolated differentially regulated by at least one of the inductors used. The analysis of the sequence of those bands revealed that only one of them displayed significant identity with genes described previously. Band-23, induced by serum and PDGF, corresponded to thrombospondin-1. Band-2 (1010 bp) (Figure 1A) recognised a transcript of 5.7 kb induced early by serum, PDGF and thrombin (Figure 1B and 1C, left-hand panel). The nucleotide sequence of band 2 shared a high level of homology with the 3'-non-translated region of members of the NGFI-B family of orphan nuclear receptors: NOR-1 (Ohkura N, Hijikuro M, Yamamoto A, Miki K. Molecular cloning of a novel thyroid/steroid receptor superfamily gene from cultured rat neuronal cells. *Biochem Biophys Res Common.* 1994; 205: 1959-1965; Hedvat CV, Irving SG. The isolation and characterisation of MINOR, a novel mitogen-inducible nuclear orphan receptor. *Mol Endocrinol.* 1995; 9: 1692-1700), Nur77 (Hazel TG, Nathans D, Lau LF. A gene inducible by serum growth factors encodes a member of the steroid and thyroid hormone receptor superfamily. *Proc Natl Acad Sci USA.* 1998; 85: 8444-8448; Milbrandt J. Nerve growth factor induces a gene homologous to the glucocorticoid receptor gene. *Neuron.* 1988; 1: 183-188) and Nurr1 (Law SW, Conneely OM, DeMayo FJ, O'Malley BW. Identification of a new brain-specific transcription factor, Nurr1. *Mol Endocrinol.* 1992 ; 6: 2129-2135).

### - Isolation of complete sequence cDNA clones

The open reading frame (ORF) of clone λSsNor32 (submitted to the GenBanK/EMBL Access N° AJ011767) codes for a polypeptide of 643 amino-acid residues which, among the receptors mentioned earlier, exhibited the greatest level of homology with NOR-1α sequences. The long 3'-non-translated region (3072 kp) of this cDNA (pNOR-1α) contains 9 copies of the sequence ATTTA, typical of short-life mRNA. The clone λSsNor83 (submitted to the GenBanK/EMBL Access N° AJ011768) exhibits a coding ORF for a polypeptide of 446 amino-acid residues (pNOR-1β) which does not contain the C-terminal region corresponding to the ligand binding domain. Alignments of the amino-acid sequence of pNOR-1α and pNOR-1β with their human homologues, rat and mouse revealed a great identity, between 89% and 94% for pNOR-1α and 87% for pNOR-1β. The greatest identity (100%) was found in the DNA binding domain. The leucines zip of the C-terminal region is conserved in three members of the NGFI-B family.

In *Northern blot* experiments, the cDNA of pNOR-α identified two transcripts (5.7 kb and 4.0 kb) temporarily induced by serum in porcine (Figure 1C, right-hand panel) and human (Figure 1D) coronary artery SMC. The induction of NOR-1 mRNA did not require the de novo synthesis of protein and was overinduced by cycloheximide (Figure 1E), as previously described for non-vascular cells.

### - Effect of growth factors, cytokines and lipoproteins on NOR-1 expression

The most powerful inductors of NOR-1 were serum, PDGF and thrombin (Figure 2). EGF and IGF-1 (only detectable in overexposed plates) also induced NOR-1. IGF-1 synergises with both PDGF and with thrombin in NOR-1 induction, though to a lesser degree than in *c-fos* induction. The effect of other mitogens, including lipopolysaccharide and angiotensin-II (data not shown) on NOR-1 was minimal.

Quiescent VSMCs were exposed to growing concentrations of low (LDL) and high (HDL) density lipoproteins obtained by means of differential centrifugation as we have described previously (Vidal F, Colomé G, Martínez-González J, Badimon L. Athergenic concentrations of native low density lipoproteins downregulate nitric oxide synthasemRMA and protein levels in endothelial cells. Eu J Biochem 1998; 252: 378-384) and the levels of NOR-1 mRNA were determined by Northern blot. The lipoproteins induced NOR-1 with a time pattern similar to that obtained with serum (data not shown).

### - Signalling pathways involved in NOR-1 induction

NOR-1 induction is dependent on the activation of protein kinase C (PKC): it was induced by PMA (a PKC activator), while GF-109203X (a PKC inhibitor) inhibited NOR-1 induction produced by serum (Figure 2A). The increase in NOR-1 expression is also dependent on Ca²⁺ mobilisation: A23817 (a calcium ionophore) induced NOR-1, while the calcium chelating agent EGTA inhibited its expression. The induction of NOR-1 produced by serum was also inhibited by a specific inhibitor of the G protein coupled receptors (pertussis toxin) and of the mitogen activated protein kinase (MAPK) (PD98059) (Figure 2A). Pertussis toxin completely abolished the induction produced by thrombin (data not shown).

By means of EMSA (Electrophoretic Mobility Shift Assay) we observed an increase in the binding of nuclear extracts coming from VSMCs stimulated with serum to a probe containing an NBRE consensus sequence (a response element to NOR-1), said increase did not take place when the activation of the PKC was inhibited (by GF10920X) or Ca²⁺ mobilisation was inhibited (by BAPTA) (Figure 3B).

### - CREB Participation in NOR-1 induction

CREB was activated by phosphorylation on Ser¹³³ in VSMCs induced with serum (Figure 4A) and nuclear extracts from human VSMCs were bound to a probe (Nor/3CRE) containing the three putative CRE elements present in the promoter of NOR-1 (from -84 to -41) (Ohkura N, Ito M, Tsukada T, Sasaki K, Yamaguchi K, Miki K. Alternative splicing generates isoforms of human neuron-derived orphan receptor-1 (NOR-1) mRNA. *Gene*. 1998; 211: 79-85) (Figure 4B). The effect was specific (competed by an excess of cold probe) and was super-shifted by antibodies against CREB but not by antibodies against c-Fos or c-Jun.

The role of the CRE elements in NOR-1 induction by serum was analysed in NIH-3T3 cells. As in human VSMCs, NOR-1 induction produced by serum in NIH-3T3 cells was inhibited when the activation of PKC or calcium mobilisation was blocked (Figure 4C). In transfection experiments the activity of NOR-1 promoter was reduced by BAPTA (a calcium chelating agent) and by GF-109203X, and was completely inhibited when the three CRE elements were mutated (Figure 4D).

### Example 2.- NOR-1 marker of atherosclerotic pathological lesions

### - NOR-1 expression in adult tissues

Heart and striated muscle express high levels of NOR-1, on the other hand low levels were detected in the thoracic aorta, lung and adrenal glands, and marginal expression (only detected in overexposed plates) in the rest of the porcine tissues analysed (Figure 5). The expression pattern of Nur77, the main member of the NGFI-B family, was similar to that of NOR-1. Nevertheless, the expression levels of Nur77 were less than those of NOR-1 in the myocardium (in relation to the levels in striated muscle) and higher in diaphragm, thoracic aorta, lung and adrenal glands.

### -NOR-1 expression in human arteries

NOR-1 mRNA levels in non-atherosclerotic arteries (coronary arteries and aorta) both in patients with DIC (dilated idiopathic cardiopathy) and with CAD (coronary artery disease) were low or undetectable (Figure 6A). NOR-1 was overexpressed in primary atherosclerotic lesions from patients with CAD. Expression of Nur77, detected in aorta but not in coronary arteries from patients with DIC, was induced in primary atherosclerotic lesions of patients with CAD though to a lesser degree than NOR-1. In human aorta arteries with aneurysm expression of both NOR-1 and Nbur77 was very high.

### -NOR-1 expression in porcine arteries dilated with balloon

Percutaneous transluminal coronary angioplasty (PTCA) temporarily induces NOR-1 mRNA in porcine coronary arteries with a pattern similar to that observed in VSMC in culture (Figure 6B). Nurr1 was also temporarily induced after PTCA (Figure 6B), but on the other hand no expression of Nur77 was detected (data not shown). By in situ RT-PCR NOR- expression was localized to SMC in the media of dilated arteries (Figure 6C).

### Example 3.- Participation of NOR-1 in the proliferation of VSMC

### - Antisense ODNs against NOR-1 inhibit DNA synthesis and cell cycle progression

Antisense sequences (AS-NOR) were directed against the two putative ATG start codons for the translation of human NOR-1 (Okhura N, Hijikuro M, Yamamoto A, Miki K. Molecular cloning of a novel thyroid/steroid receptor superfamily gene from cultured rat neuronal cells. *Biochem Biophys Res Common.* 1994; 205: 1959-1965; Hedvat CV, Irving SG. The isolation and characterisation of MINOR, a novel mitogen-inducible nuclear orphan receptor. *Mol Endocrinol.* 1995; 9: 1692-1700). Both antisense ODNs effectively inhibited the incorporation of [³H] thymidine in human VSMCs in a dose-dependent manner. Figure 7A shows the effect of AS₁-NOR sequences. The sense NOR-1 ODNs produced no effect. A similar inhibiting effect was produced by growing concentrations of an antisense ODN against *c-fos* (data not shown), previously used for analysing the role of this proto-oncogene in cell proliferation (Leon T, Vazquez E, Sanz C, Vega JA, Mato JM, Giraldez F, Represa J, Varela-Nieto I., Insulin-like growth factor-I regulates cell proliferation in the developing inner ear, activating glycosyl-phosphatidylinositol hydrolysis and Fos expression. *Endocrinology.* 1995; 136: 3494-3503). The levels of NOR-1 mRNA in cells treated with antisense ODNs were significantly lower than in control cells or in those treated with the sense NOR-1 sequences or with anti*-c-fos* ODNs (Figure 7B).

Flow cytometry analysis of the distribution of the phases of the cell cycle revealed a significant reduction of entry into S phase following stimulation with serum (40% and 42% with AS₁- and AS₂-NOR respectively) and a parallel increase in the cells in G1 phase. Figure 7C shows representative FACS profiles corresponding to human VSMC with and without AS₁-NOR. Sense NOR-1 ODNs did not produce any effect on the cell cycle distribution. A similar effect was produced by antisense ODNs against *c-fos* (41% reduction in the entrance of cells into S phase and a similar increase in the number of cells in G1 phase) .

### - Antisense ODNs against NOR-1 inhibit the growth of human VSMCs induced by lesion

In an *in vitro* system previously validated for lesion repair (Lowe HC, Fahmy RG, Kavuna MM, Baker A, Chesterman CN, Khachigian LM, Catalytic oligodeoxynucleotides define a key regulatory role for each growth response factor-1 in the porcine model of coronary in-stent resenosis. *Cir Res.* 2001; 89: 670-677) AS₁- or AS₂-NOR significantly inhibited the invasion of the denuded zone by 45% and 46% respectively (Figure 7D). On the other hand, equivalent concentrations of sense NOR-1 ODNs did not modulate this process. The effect produced by the ODNs against NOR-1 was similar to that produced by the ODNs against *c-fos.* Figure 7E shows a representative image of human VSMCs subjected to this process in the presence of AS₁-NOR.

### Example 3.- The NOR transcription factor is induced in monocytes/macrophages activated by mitogenic factors

Human monocytes isolated by means of elutriation (Sanderson RJ et al. Isolation and enumeration of peripheral blood monocytes. J Immunol 1977; 118: 1409-1414) were incubated for 24 hours in multi-well plates and were then exposed for one hour to different inductors [human serum, PDGF-BB, thrombin, and a PKC inductor (PMA)] and NOR-1 mRNA levels were determined by means of RT-PCR as has been previously stated (Figure 8).

### Example 4. NOR-1 is induced by lipoproteins in human endothelial cells and by growth factors in human monocytes/macrophages.

### - NOR-1 is induced in endothelial cells by low density lipoproteins (LDL) and mitogenic factors.

Growing concentrations of low density lipoproteins (LDL), but not of high density lipoproteins (HDL), induced levels of NOR-1 mRNA in human umbilical vein endothelial cells (HUVEC) (Figure 8A). In VSMC, both LDL and HDL induced the expression of NOR-1 (Figure 8B). 10% serum and thrombin (10 U/mL) also induced NOR-1 expression both in HUVEC and in porcine aorta artery endothelial cells (PAEC) (Figure 8C). In these experiments endothelial cells in a state of semi-confluence, cultured in low serum containing medium, were induced with growing concentrations of lipoproteins or other inductors for 1 h.

### - NOR-1 is induced by growth factors in human monocytes/macrophages in culture

Human monocytes were incubated for 24 hours in multi-well plates and were then exposed for 1 h to different inductors [10% human serum, 5% nmol/L PDGF-BB, 10 U/mL of thrombin, and a PKC inductor (PMA, 5 µmol/L)] and NOR-1 mRNA levels were determined by RT-PCR. Figure 9 shows the expression levels of NOR-1 achieved in these cells exposed to different inductors.
In the following section on Materials and Methods, the cells, plasmids, antisense sequences, oligonucleotides and animals used in said Examples are identified, along with the methods for evaluating the expression of proteins of interest in this present invention NOR-1 and Nur77 (Northern blot, *in situ* RT-PCR, EMSA), western blot, the angioplasty technique, VSMC lesion technique and statistical analysis.

### Materials and Methods

### Cell culture

The VSMCs were obtained by the explant technique starting from human and porcine coronary arteries (Martínez-González J, Viñals M, Vidal F, Llorente-Cortés V, Badimon L, Mevelonate deprivation impairs IGF-I/Insulin signalling in human vascular smooth muscle cells. *Atherosclerosis* 1997; 135: 213-223). Cells were cultured as described earlier and in the experiments cells of between the 2^{nd} and 5^{th} passage were used. The cells were sown on multi-well plates and when they reached confluence, quiescence was induced by incubating them for 48 hours in a medium with 0.4% foetal calf serum (FCS). Quiescent cells were stimulated with 10% human serum or with 5 nmol/L of Platelet-Derived Growth Factor-BB (PDGF-BB) (Amersham-Pharmacia), 10 U/mL of α-thrombin (Diagnostica-Stago), 10 nmol/L of Insulin-like Growth Factor-1 (IGF-1) (Sigma), 1 µmol/L of insulin (Novo-Nordisk), 10 nmol/L of Epidermal Growth Factor (EGF) (Amersham-Pharmacia), 25 nmol/L of basic Fibroblast Growth Factor (bFGF) (Amersham-Pharmacia), 5 nmol/L of Tumour Necrosis Factor-α (TNF-α) (Amersham-Pharmacia), 5000 U/mL of interleucine-1β (IL-1β) (Amersham-Pharmacia), 0.1 nmol/L of Transforming Growth Factor beta (TGF-β) (Amersham-Pharmacia) or by growing concentrations of A23187 (Alexis) and 4-β-phorbol-12-myristate-13-acetate (PMA) (Sigma) in different experiments. When inhibitors were used, the VSMCs were preincubated for 30 minutes before the stimulus. The inhibitors used were: 1 µg/mL of pertussis toxin (Sigma), 50 µmol/L of PD98059 (Sigma), 15 µmol/L of 1,2-bis(2-aminophenoxy)ethane-N,N,N',N'-tetrakis tetraacetic acid acetoxymethyl ester (BAPTA-AM) (Sigma) or growing concentrations of GF-109203X (Sigma) or EDTA.

Human umbilical vein endothelial cells (HUVEC) and porcine aorta artery endothelial cells (PAEC) were obtained by means of the digestion method with collagenase and cultured as we have previously described (Martínez-González J, Raposo B, Rodriguez C, Badimon L, 3-hydroxy-3-methylglutaryl coenzyme A reductase inhibition prevents endothelial NO synthase downregulation by atherogenic levels of native LDLs. Arterioscler Thromb Vasc Biol 2001; 21: 804-809).

Human monocytes were obtained by means of elutriation starting from concentrates of white cells in accordance with the methodologies previously described (Sanderson RJ et al. Isolation and enumeration of peripheral blood monocytes. J Immunol 1977; 118: 1409-1414).

The inhibitors did not produce any effect on the cell morphology, cell apoptosis (analysed by staining with Hoesch 33258 dye) or on cell viability analysed with a commercial kit for mitochondrial dehydrogenase activity (*XTT based assay for cell viability,* Roche).

### mRNA-DD analysis

Total RNA from VSMC coming from the tunica media of porcine coronary arteries was isolated, quiescent or stimulated for 1 hour with 10% human serum, 10 U/mL of α-thrombin or 5 nmol/L of PDGF-BB using Quick-Prep™ (Amersham-Pharmacia), *mRNA-DD* analysis was conducted with the Delta™ RNA fingerprinting kit (Clontech) as described (Rodriguez C, Martínez-González J, Sánchez-Gómez S, Badimon L. LDL downregulate CYP51 in vascular endothelial cells in the arterial wall through a SREBP-2 dependent mechanism. *Circ Res.* 2001; 88: 268-274). Differential bands were cloned in the pGEM-T™ vector (Promega) and sequenced with the ABIPrism dRhodamine Terminator Sequencing kit (Perkin Elmer).

### Analysis of the cDNA library

Band number 2 identified by *mRNA-DD* was used as a probe for the analysis of a cDNA library in λZAPII (Stratagene) prepared from porcine coronary artery SMC mRNA (stimulated with human serum for 1 hour). In an analysis of 1 x 10⁶ colonies, 101 positive clones were identified, which were characterised by mapping with restriction enzymes. Clones λSsNor32 and λSsnor83 were subcloned for later analysis. The nucleotide sequence was obtained by automatic sequencing of both strands of cDNA. The multiple alignments of protein were obtained using the ClustaIW service from the European Bioinformatics Institute.

### Analysis by Northern blot

Total RNA was obtained as described above and analysed by *Northern blot* as previously described (Martínez-González J, Viñals M., Vidal F, Llorente-Cortés V, Badimon L, Mevelonate deprivation impairs IGF-I/Insulin signalling in human vascular smooth muscle cells. *Atherosclerosis* 1997; 135: 213-223). As probes NOR-1α cDNA, a *c-fos* rat cDNA and a ribosomal cDNA (for normalising the membranes) were used.

### Electrophoretic Mobility Shift Assay (EMSA)

Nuclear extracts (3 µg) from human VSMC were used along with double chain probes corresponding to the human NOR-1 promoter sequence (from -84 to -41; Nor/3CRE), which contains the three CRE sites or a probe containing an NBRE consensus sequence, as described (Rodriguez C, Martínez-González J, Sánchez-Gómez S, Badimon L. LDL downregulate CYP51 in vascular endothelial cells in the arterial wall through a SREBP-2 dependent mechanism. *Circ Res.* 2001; 88: 268-274). The super-shift experiments were conducted using antibodies against CREB, c-Fos and c-Jun (Santa Cruz Biotechnology).

### Western blot analysis

CREB levels were analysed by Western blot (of the phosphorylated and dephosphorylated forms) in cell extracts coming from VSMC (Rodriguez C, Martínez-González J, Sánchez-Gómez S, Badimon L. LDL downregulate CYP51 in vascular endothelial cells in the arterial wall through a SREBP-2 dependent mechanism. *Circ Res.* 2001; 88: 268-274) using specific antibodies (Santa Cruz Biotechnology).

### Construction of plasmids containing the NOR-1 promoter

The plasmid pNORα/-1703 which contains human NOR-1 promoter (from -1703 to +264) was kindly provided by Dr N Ohkura. Directed mutagenesis on the three possible CRE sites present in the NOR-1 promoter was carried out by QuickChange™ Site-Directed Mutagenesis Kit (Stratagene). The changes introduced in the sequence of the mutant construction [pNORα/-1703-(M)] were (-79) TGcatcAGCGTCCCATGcatgCACATTGcgtaCT(-46) (SEQ ID NO1) (changes are indicated by letters in lower case). The new sequence was analysed by means of promoter analysis programs in order to confirm that no new response elements had been created.

### Transient transfection and luciferase assay

NIH-3T3 cells were transfected with luciferase expression vectors using the Nucleofactor™ system (Amaxa Biosystems), following the manufacturer's specifications. Quiescence was induced in the transfected cells incubating them for 36 hours in a medium with 0.4% FCS and they were then stimulated with serum for 4 hours. Luciferase activity was determined in cell lysates with a luminometer. pSVβ-gal (Promega) was used as an internal control.

### Samples of human arteries

Samples of aortas and coronary arteries were obtained from transplanted hearts coming from human patients with idiopathic cardiomyopathy (DIC) or CAD. Coronary arteries were examined at low magnification and classified in two categories: areas with atherosclerotic lesions and areas without atherosclerotic lesions. The present study and all human samples followed the internal directives and were approved by the Ethical Committee of the Hospital of Santa Creu i Sant Pau.

### Angioplasty in the porcine model

Yorkshire-Albino pigs were selected (body weight 30-35 kg) to carry out the angioplasties. The handling, upkeep and care of the animals, along with the procedures carried out in this study, were done following the institutional directives and also the directives for animal research of the American Heart Association. The methodology for the angioplasty with coronary balloon has been widely described.¹³ The coronary lesions were produced by inflating a balloon catheter (Cordis) of 4.0x20.0 mm three times at 8 atm. Once the angioplasty was finished, the animals were slaughtered after 1, 2, 4 and 6 hours (n=20). This angioplasty procedure produces an over-extension of the artery causing severe damage.

### gene expression analysis in human and porcine arteries

NOR-1, Nur77 and Nurr1 expression was analysed by RT-PCR. The specific oligonucleotides used were NOR-1 5'-AGGG CTGCAAGGGCTTTTTCAAGA GA-3' (SEQ ID NO2) and 5'-TGCT TTCTACAGGAGCTGCT-3' (SEQ ID N03); Nur77, 5'-CCACAGCCTCCAGCCTTTT-3' (SEQ ID NO4) and 5'-TTTCGCCGCCTCTTGTCCAC -3' (SEQ ID NO5) ; Nurr1, 5'-CTCCCGCCTCTCCCTCTTCT-3' (SEQ ID NO10) and 5'-TTGGTCGGCTTTGGGCAGGT -3' (SEQ ID NO11). The levels of glyceraldehyde-3-phosphate (GAPDH) were used for normalising the results (Rodríguez C, Martínez-González J, Sánchez-Gómez S, Badimon L. LDL downregulate CYP51 in vascular endothelial cells in the arterial wall through a SREBP-2 dependent mechanism. *Circ Res.* 2001; 88: 268-274). The amplification was carried out at 24 (NOR-1, Nur77 and Nurr1) or 22 (GAPDH) cycles: denaturing, 94°C for 30 s: annealing at 61°C (NOR-1, Nurr1 and GAPDH) or 55°C (Nur77) for 1 minute and polymerisation, 72°C for 1 min. 30 s. PCR products stained with dioxygenine were resolved in agar gels, transferred to nylon membranes (Scheicher & Schuell) and detected as described (Rodriguez C, Martínez-González J, Sánchez-Gómez S, Badimon L. LDL downregulate CYP51 in vascular endothelial cells in the arterial wall through a SREBP-2 dependent mechanism. *Circ Res.* 2001; 88: 268-274).

### In situ RT-PCR

*In situ* PCR was conducted on specimens subjected to PTCA using the EZ rTth RNA PCR Kit™ (Perkin Elmer) according to the manufacturer's protocol. The primers used for amplifying the mRNA were: 5'-GATGCTATCCCAGCAGGAAA-3' (SEQ ID NO 8) and 5'-CATCAAGAACGGCCAAAAT-3' (SEQ ID N09). The amplification was carried out for 25 cycles and the specific annealing conditions were 55°C for 90 s. The PCR products stained with dioxygenine were detected using a system coupled to alkaline phosphatase and NBT/BCP.

### DNA synthesis and distribution of the cell cycle determination

Quiescent SMCs from human coronary arteries were induced with 10% human serum in culture medium with 0.5 µCi/mL [³H] thymidine in presence or absence of growing concentrations of phosphorothioated oligodeoxynucleotides (ODNs) and incorporation of [³H] thymidine was determined as has been previously described (Martínez-González J, Viñals M., Vidal F, Llorente-Cortés V, Badimon L, Mevelonate deprivation impairs IGF-I/Insulin signalling in human vascular smooth muscle cells. *Atherosclerosis* 1997; 135: 213-223). The effect of ODNs on the cell cycle was analysed using flow cytometry as has been described (Darzinkiewic Z, Juan G. Nucleic acid analysis. In: Robinson JP. ed. *Current Protocols in Cytometry.* New York, John Wiley and Sons, Inc. 1997: 1-24). Antisense ODNs used were: antisense-1 NOR-1 (AS₁-NOR; 5'-TTGGACGCAGGGCAT-3') (SEQ ID NO 6) and antisense-2 NOR-1 (AS₂-NOR; 5'-GCTGCCAAGGTCCAT-3') (SEQ ID NO 7), complementary to nucleotides 732 to 746 and 849 to 863 of human NOR-1 mRNA (access N° NM-006981) respectively. As controls, the corresponding ODNs sense and antisense sequences against *c-fos* were used, as previously used for analysing the role of this proto-oncogene in cell proliferation (Leon Y, Vazquez E, Sanz C, Vega JA, Mato JM, Giraldez F, Represa J, Varela-Nieto I. Insulin-like growth factor-I regulates cell proliferation in the developing inner ear, activating glycosyl-phosphatidylinositol hydrolysis and Fos expression. *Endocrinology.* 1995; 136: 3494-3503).

### In vitro VSMC lesion

The capacity of antisense ODNs to inhibit tissue repair of VSMCs following mechanical lesion was analysed in human coronary artery SMCs in culture as has been previously described (Lowe HC, Fahny RG, Kavuna MM, Baker A, Chesterman CN, Khachigian LM, Catalytic oligodeoxynucleotides define a key regulatory role for each growth response factor-1 in the porcine model of coronary in-stent resenosis. *Cir Res.* 2001; 89: 670-677). Briefly, quiescent human coronary artery SMCs in a state of confluence were scraped and then incubated for 72 h at 37°C in presence and absence of the previously stated ODNs. Cells were fixed and stained with methylene blue. Images were digitised by means of a Sony 3CCD camera and the number of cells in the denuded zone was determined.

### Statistical analysis

The results were expressed as the mean±EEM. The Stat View^{TN} II program (Abacus Concepts) for Macintosh computer was used for the analysis. Multiple groups were analysed by means of an ANOVA factor, following the Fisher PLSD test to determine specific differences among groups.

LIST OF SEQUENCES
<110>  SPANISH HIGHER COUNCIL OF SCIENTIFIC RESEARCH

<120>  NOR-1 (neuron-derived orphan receptor-1), novel
diagnostic and therapeutic target for cardiovascular diseases

<130>  NOR-1

<160>  11

<170>  PatentIn version 3.1


<210>  1
<211>  26
<212>  DNA
<213>  Artificial Sequence
<220>
<223>  Oligo NOR-1

<400>  1
agggctgcaa gggcttttc aagaga                                    26


<210>  2
<211>  20
<212>  DNA
<213>  Artificial Sequence

<220>

<223>  Oligo NOR-1b

<400>  2
tgctttctac aggagctgct                                          20

<210>  3
<211>  19
<212>  DNA
<213>  Artificial Sequence

<220>

<223>  Oligo Nur77 a

<400>  3

ccacagcctc cagcctttt 19

<210> 4
<211> 20
<212> DNA
<213> Artificial Sequence

<220>

<223> Oligo Nur77 b

<400> 4
tttcgccgcc tcttgtccac 20

<210> 5
<211> 34
<212> DNA
<213> Artificial Sequence

<220>

<223> pNORa/-1703-(M) sequence

<400> 5
tgcatcagcg tcccatgcat gcacattgcg tact 34

<210> 6
<211> 15
<212> DNA
<213> Artificial Sequence

<220>

<223> NOR-1 antisense-1 oligo
<400> 6
ttggacgcag ggcat 15

<210> 7
<211> 15
<212> DNA
<213> Artificial Sequence

<220>

<223> NOR-1 Antisense-2
<400> 7
gctgccaagg tccat 15

<210>  8
<211>  20
<212>  DNA
<213>  Artificial Sequence

<220>

<223> NOR-1 oligo in situ RT-PCR a

<400>  8
gatgctatcc cagaggaaa                                                    20


<210>  9
<211>  19
<212>  DNA
<213>  Artificial Sequence

<220>

<223> NOR-1 oligo in situ RT-PCR a

<400>  9
catcaagaac ggccaaaat                                                    19


<210>  10
<211>  20
<212>  DNA
<213>  Artificial Sequence

<220>

<223> Nurr1 oligo a

<400>  10
ctcccgcctc tccctcttct                                                   20


<210>  11
<211>  20
<212>  DNA
<213>  Artificial Sequence

<220>

## Claims

1. Identification procedure of NOR-1 and/or Nu77 and/or Nurr1 in biological samples from mammals, preferably humans, for diagnosis and follow-up of cardiovascular diseases associated with NOR-1 activation or for monitoring, following-up or assessing the effectiveness of pharmacological treatments for said cardiovascular diseases, **characterised in that** it comprises the following parts:
a) Obtaining the biological sample by means of blood extraction or intravascular surgery, and
b) Identification of NOR-1 and/or Nur77 and/or Nur1 in said biological sample, where a positive or increased result with respect to a base level considered physiological, will indicate the existence of a cardiovascular disease associated with activation proteins of the NGFI-B family, or where the comparison of NOR-1 levels determined over time would indicate development of the cardiovascular disease or whether, for example, the treatment administered for that disease shows any therapeutic effect.

2. Identification procedure of NOR-1 and/or Nur77 and/or Nur1 according to claim 1 **characterised in that** NOR-1 identification is done by determination of the expression levels of human NOR-1, in any of its two alpha and beta isoforms, and/or of Nur77 and/or of Nurr1, be it its messenger RNA or the corresponding proteins.

3. Identification procedure of NOR-1 and/or Nur77 and/or Nur1 according to claim 1 **characterised in that** the biological sample consists of body samples such as blood (especially its constituent cells), vascular cells and also peripheral tissue samples.

4. Identification procedure of NOR-1 according to any of claims 1 to 3, **characterised in that** the methods used for the determination of the mRNA levels belong, among others, to the following group: RT-PCR, Northern blot, DNA arrays or biochips of DNA or oligonucleotides, in situ hybridation or *in situ* RT-PCR; and because the methods for determining protein levels are based on polyclonal or monoclonal antibodies which recognise NOR-1 and/or Nur77 and/or Nurr1 and belong to the following group, among others: Western blot, immunocytochemistry, immunohistochemistry and protein biochips.

5. Identification procedure of potential NOR-1 activity modulating compounds **characterised by** the following stages:
a) placing said compound in contact within a biological environment where the functionally active protein NOR-1 exists,
b) determination of NOR-1 protein expression or activity levels, and
c) selection of said compound as NOR-1 modulating compound (or of other members of the NGFI-B family) if the inhibition or boosting of NOR-1 effects is noted in the presence of the compound.

6. Identification procedure of potential NOR-1 activity modulating compounds according to claim 5 **characterised in that** the biological environment is a VSMC culture, endothelial cells and monocytes/macrophages where in the presence or otherwise of NOR-1 - serum, thrombin, PDGF and EGF, among others - the potential compound to be assessed is added and a determination of expression or activity levels of NOR-1 is then made.

7. Identification procedure of potential NOR-1 activity modulating compounds of according to claim 5 **characterised in that** the biological environment is an animal model, preferably porcine, which is subjected to a percutaneous transluminal coronary angioplasty (PTCA) operation, and is administered with the potential compound to assess.

8. NOR-1 inhibitor or booster compound (or of other members of the NGFI-B family) **characterised in that** it is identified by means of a procedure according to any of claims 5 to 7.

9. Pharmaceutical composition **characterised in that** it comprises a therapeutically effective quantity of a inhibitor or booster compound of NOR-1 (or of other members of the NGFI-B family) according to claim 8.

10. Use of a pharmaceutical composition according to claim 9 in the preparation of a medicine for the treatment of cardiovascular diseases associated with NOR-1 activation in humans.

11. Gene therapy procedure for the treatment and prevention in humans of cardiovascular diseases associated with NOR-1 activation, **characterised by** blocking NOR-1 expression in cells implicated in the aetiopathogeny of those diseases, for example by means of expression of NOR-1 antisense oligonucleotides (ODNs), or nucleotide sequences directed at blocking NOR-1 expression, using vectors or carriers that permit the entrance of said sequences into the cells. (for example, coated "stents" among others) .

12. Gene therapy procedure for the treatment and prevention in humans of cardiovascular diseases associated with NOR-1 activation, according to claim 11, **characterised in that** the antisense ODNs/against NOR-1 are the following: AS1-NOR-1 (SEQ ID NO6) and AS2-NOR-1 (SEQ ID NO 7).
